# EUROPEAN PATENT APPLICATION

(11) **EP 3 831 922 A1**
(43) Date of publication of application: **09.06.2021**
(21) Application number: 20211438.5
(22) Date of filing: 03.12.2020
(51) Int. Cl.: C12M 1/24, C12M 1/00, C12M 3/00, C12M 3/04

(54) **ROLLER TUBE CULTURE SYSTEM**

(30) Priority: 04.12.2019 US 201916702967
(71) Applicant: Scientific Industries, Inc., Bohemia, New York 11716 (US)
(72) Inventor: Cremonese, Joseph G., Greensburg, Pennsylvania 15601-5720 (US)
(74) Representative: Fritzsche, Thomas

(57) **Abstract**

Embodiments relate to systems and methods for increasing the cell growth surface within the roller tubes used as reaction vessels in a cell culture system by using a rotating platform or cage-like structure with a center of rotation of each of roller tubes being held therein being coaxial with the circumferential distribution of the plurality of roller tubes held in the platform. Each of the roller tubes has a superellipse or squircle cross-sectional shape to further increase the cell growth surface within the roller tubes. Additional improvements include use of a removable cell adherence liner, a cell perfusion system, a cell culture monitoring system, and a gas circulation system.

## Description

### BACKGROUND OF THE INVENTION

### Field of the Invention

Embodiments relate to systems and methods for increasing the cell growth surface within the roller tubes used as reaction vessels in a cell culture system. Additional improvements include use of a removable cell adherence liner, a cell perfusion system, a cell culture monitoring system, and a gas circulation system.

### Background of the Related Art

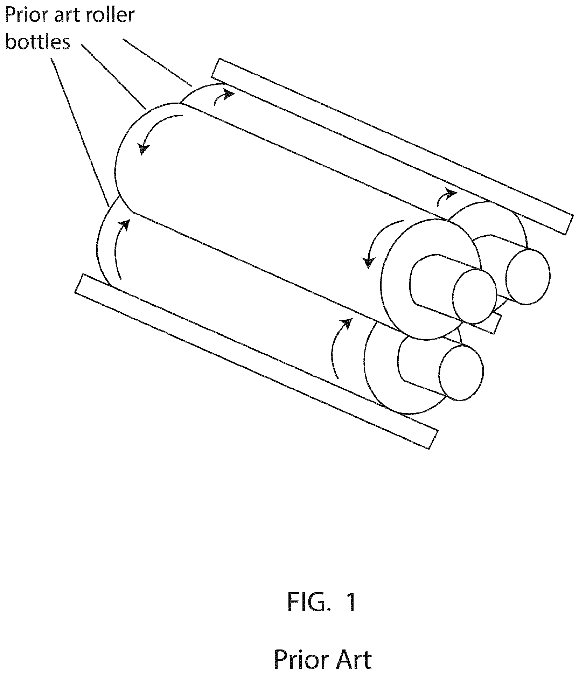

Roller bottles used as reaction vessels for adherent cell culture techniques have been in use for a bit longer than forty years. FIG. 1 shows an exemplary conventional roller bottle set-up. This set-up has three roller bottles arranged in a parallel manner whereby rotating at least one of them causes all three to rotate. The cells within each bottle adhere to the inner surface walls, and a volume of liquid cell growth media rests on a bottom surface of each roller bottle. As each bottle rotates, the cells that are adhered to the inner surface travel into and out from the media. As can be seen form FIG. 1, each bottle is rotated about a central axis that runs along the longitudinal axis of the length of the bottle. The most recent 20 years has demonstrated that usage of roller bottles has increased because of the success of growing adherent cells in roller bottles. Improved culture medium in combination with improved treatment of adherent growth surfaces in roller bottles has contributed to the success of growing adherent cells. Additionally, gas transfer is more efficient in roller bottles than in T-Flasks because of a large contact area between the gaseous phase and the thin liquid-medium film on the wall of the horizontally incubated roller bottles.

Such conventional roller bottle designs, however, suffer from technical problems, especially when the use of advanced culture methods are desired. For instance, scale-up can be achieved by adding more roller bottles, but space requirements for that type of scale-up quickly becomes prohibitive. In addition, increasing the physical size of each roller bottle is problematic. In a 12 cm. diameter bottle, a growth area increase by a factor of 10 would require the new bottle to have a diameter of 1.2 meters which is highly improbable and impractical. Increasing the size of a roller bottle also increases the dead space within the bottle. Adding ribs or pleats to the surface of or within the surface of the inside walls of each roller bottle so as to form them around the central axis of the roller tube can result in a doubling of the cell attachment area, but harvesting cells from pleated or ribbed bottles is a tedious task.

Consequently, conventional roller bottles cannot be configured for automated, advanced perfusion studies. In addition, there is currently no unencumbered way to monitor real-time pH, CO₂ levels, and/or dissolved oxygen (DO) in a roller bottle rotating around a central axis.

The present invention will overcome at least one of the technical problems identified above.

### BRIEF SUMMARY OF THE INVENTION

Embodiments of the systems and methods disclosed herein relate to meaningfully increasing the cell growth surface within roller tubes versus roller bottles, without increasing the cubical space requirement (i.e., the volume of space occupied by all of the tubes) by using a rotating platform that holds the tubes as the platform is rotated. Instead of each roller tube being rotated on a central axis along the longitudinal axis of the tube (i.e., the rotation is coaxial with the growth surface), all of the tubes are rotated in unison as the platform is rotated so that the center of rotation of each of the roller tubes is coaxial with the circumferential distribution of the plurality of roller tubes held in the platform. This rotating platform configuration allows for an increase in the total circumferential surface area available for cell growth by reducing the size of the roller tubes and increasing the number of roller tubes. Consequently, the combined growth surface area can be increased without increasing the cubical space requirement.

In addition, each roller tube can have a superellipse cross-sectional shape, which can include a squircle (a shape intermediate between a square and a circle -e.g., a square with rounded edges). This can be done to increase the circumferential surface area available for cell growth within each roller tube.

In addition, each roller tube can have a cell adherence liner that allows for cell adherence and growth. The liner can be easily removed from the roller tube. Once the cell adherence liner is removed, the cells can be collected.

The roller tube culture system includes a cell perfusion system to facilitate retention of cells inside the roller tube while simultaneously removing cell waste products and media depleted of nutrients by cell metabolism. The cell perfusion system can be configured to provide fresh media to the cells at the same rate as the spent media is removed. The cell perfusion system includes a perfusion assembly (e.g., an arrangement of tubing, seals, and valves) connected to or within each roller tube that can be placed into fluid communication with a media exchanger tube (e.g., a tube having a bladder or bag within that supplies fresh media to cell growth vessels and a volume of space between the inside of the tube and the bladder to receive spent media from the cell growth vessels). Some embodiments of the media exchanger tube can be configured to facilitate co-culturing via trans-mixing.

The roller tube culture system can include a cell culture monitoring system. The cell culture monitoring system includes a sensor patch and a reader to facilitate real-time pH and dissolved oxygen monitoring.

The roller tube culture system can include a gas circulation system configured to adjust the carbon dioxide (CO₂) and dissolved oxygen (dissolved O₂ or DO) levels in the roller tube(s). With conventional systems, if the pH, CO₂ and dissolved O₂ levels were trending to undesirable levels, the media would have to be replaced. With the inventive system, the levels of CO2, for pH control, and dissolved O₂ can be adjusted instead of replacing the media. This can be achieved by the gas circulation system introducing and/or removing gas from the incubation environment of the roller tube(s). The introduction and/or removal of gas can include gas suitable for cell growth, such as oxygen, carbon dioxide, nitrogen, or a mixture of these gases.

In an exemplary embodiment, a platform for a cell culture system can include a framework configured to rotate about an axis of rotation, the framework configured to receive and retain a plurality of reaction vessels so as to be arranged to position each reaction vessels adjacent to the axis of rotation.

In some embodiments, the framework is configured to arrange the plurality of reaction vessels circumferentially about the axis of rotation.

In some embodiments, the framework is configured to arrange the plurality of reaction vessels so that a longitudinal axis of each reaction vessel is parallel with the axis of rotation.

In an exemplary embodiment, a platform for a cell culture system can include a framework configured to rotate about an axis of rotation, the framework configured to receive and retain a plurality of reaction vessels so as to cluster the plurality of reaction vessels around the axis of rotation.

In some embodiments, when the framework is rotated about the axis of rotation, the plurality of reaction vessels revolves about the axis of rotation.

In some embodiments, the framework is further configured to receive and retain at least one media reservoir. The framework is further configured to cluster the plurality of reaction vessels and the at least one media reservoir around the axis of rotation.

In some embodiments, when the framework is rotated about the axis of rotation, the plurality of reaction vessels and the at least one media reservoir revolve about the axis of rotation.

In an exemplary embodiment, a reaction vessel can include a roller tube having a roller tube first end, a roller tube second end, and an elongated body, wherein the roller tube has a superellipse cross sectional shape.

In some embodiments, the superellipse cross sectional shape is a squircle.

In an exemplary embodiment, a reaction vessel can include a roller tube having a roller tube first end, a roller tube second end, and an elongated body. The reaction vessel has a first end cap configured to be removably attached to the roller tube first end, the first end cap having a first inlet/outlet opening and a first vent aperture. The reaction vessel has a second end cap configured to be removably attached to the roller tube second end, the second end cap having second inlet/outlet opening and a second vent aperture. The reaction vessel has tubing extending from the first inlet/outlet opening to the second inlet/outlet opening, the tubing configured to controllably release media and/or gas.

In some embodiments, the first vent aperture does not subtend the second vent aperture.

In an exemplary embodiment, a cell adherence liner for a reaction vessel can include a liner having a liner first side and a liner second side, the liner first side being treated to facilitate cell adherence and cell growth, and the liner second side being configured to exhibit surface attraction to material of the reaction vessel to facilitate removable attachment to the reaction vessel and/or having an adhesive applied thereto to facilitate removable attachment to the reaction vessel.

In some embodiments, the liner first side is corona treated and/or plasma etched.

In some embodiments, the liner is fluorinated ethylene propylene (FEP) polytetrafluoroethylene (PTFE).

In an exemplary embodiment, a media exchanger vessel can include a container having a cavity and a bladder within the cavity, a volume of space between an inner surface of the container and the bladder, an inlet nozzle configured to facilitate introduction of spent media into the container, and an outlet nozzle configured to facilitate flow of fresh media from the bladder.

In some embodiments, the inlet nozzle is in fluid communication with the volume of space between the inner surface of the container and the bladder.

In some embodiments, the inlet nozzle is in fluid communication with the bladder.

In an exemplary embodiment, a roller tube culture system can include a rotating platform. The rotating platform has a framework configured to rotate about an axis of rotation, the framework configured to receive and retain a plurality of reaction vessels and at least one media reservoir so as to cluster the plurality of reaction vessels and the at least one media reservoir around the axis of rotation. Each reaction vessel includes a roller tube having a roller tube first end, a roller tube second end, and an elongated body, wherein the roller tube has a superellipse cross sectional shape. Each reaction vessel has a first end cap configured to be removably attached to the roller tube first end, the first end cap having a first inlet/outlet opening and a first vent aperture. Each reaction vessel has a second end cap configured to be removably attached to the roller tube second end, the second end cap having second inlet/outlet opening and a second vent aperture. Each reaction vessel has tubing extending from the first inlet/outlet opening and terminating by threading onto a solid pin positioned in the second inlet/outlet opening, the tubing configured to controllably release media and/or gas into the roller tube reaction space. Each reaction vessel has a cell adherence liner. The cell adherence liner includes a liner having a liner first side and a liner second side, the liner first side being treated to facilitate cell adherence and cell growth, and the liner second side being configured to exhibit surface attraction to material of the reaction vessel to facilitate removable attachment to the reaction vessel and/or having an adhesive applied thereto to facilitate removable attachment to the reaction vessel. The at least one media reservoir includes a container having a cavity and a bladder within the cavity, wherein a volume of space can exist between an inner surface of the container and the bladder. An inlet nozzle is provided and configured to facilitate introduction of spent media into the container. An outlet nozzle is provided and configured to facilitate flow of fresh media from the bladder.

In some embodiments, the roller tube culture system includes a cell culture monitoring system.

In some embodiments, the roller tube culture system includes a gas circulation system.

Further features, aspects, objects, advantages, and possible applications of the present invention will become apparent from a study of the exemplary embodiments and examples described below, in combination with the Figures, and the appended claims.

### BRIEF DESCRIPTION OF THE DRAWINGS

The above and other objects, aspects, features, advantages and possible applications of the present invention will be more apparent from the following more particular description thereof, presented in conjunction with the following drawings, in which:
FIG. 1 shows a conventional roller tube set-up.
FIG. 2 shows a cross section of a conventional roller bottle juxtaposed with embodiments of the inventive roller tube and rotating platform.
FIG. 3 shows a conventional roller tube.
FIG. 4 shows an exemplary platform and roller tube arrangement that can be used with the roller tube culture system.
FIG. 5 shows an exemplary roller tube with an exemplary cell adherence liner that can be used with the roller tube culture system.
FIG. 6 shows an exemplary cell perfusion system that can be used with the roller tube culture system.
FIG. 7 shows another exemplary platform and roller tube arrangement that can be used with the roller tube culture system.
FIG. 8 shows an exemplary media exchanger tube configuration that can be used with the roller tube culture system.
FIG. 9 shows another exemplary media exchanger tube configuration that can be used with the roller tube culture system.
FIG. 10 shows an exemplary cell culture monitoring system that can be used with the roller tube culture system.
FIG. 11 shows another exemplary platform and roller tube arrangement that can be used with the roller tube culture system.
FIG. 12 shows an exemplary gas circulation system and incubator system that can be used with the roller tube culture system.

### DETAILED DESCRIPTION OF THE INVENTION

The following description is of an embodiment presently contemplated for carrying out the present invention. This description is not to be taken in a limiting sense, but is made merely for the purpose of describing the general principles and features of the present invention. The scope of the present invention should be determined with reference to the claims.

Embodiments of the roller tube culture system 100 can include a rotating platform 102. The rotating platform 102 can be configured to retain a plurality of roller tubes 104 (e.g., reaction vessels). The platform 102 can be configured as a framework, stage, a cage, a scaffold, etc. that has means to receive and retain a plurality of roller tubes 104 so as to arrange them in juxtaposition to each other and so that they are arranged in a parallel manner. For instance, each roller tube 104 can be a container having a roller tube first end 106, a roller tube second end 108, and an elongated body 110 with a longitudinal axis 112 running from the roller tube first end 106 to the roller tube second end 108. Using four roller tubes 104 as an example, the platform 102 can be configured to receive and retain a first roller tube 104, a second roller tube 104 so that it is adjacent the first roller tube 104 and its longitudinal axis 112 is parallel with that of the first roller tube 104, a third roller tube 104 so that it is adjacent to both the first and second roller tubes 104 and its longitudinal axis 112 is parallel with those of the first and second roller tubes 104, and a fourth roller tube 104 so that it is adjacent the first, second, and third roller tubes 104 and its longitudinal axis 112 is parallel with those of the first, second, and third roller tubes 104. Use of four roller tubes 104 is for exemplary purposes only and it is understood that more or less roller tubes 104 can be used.

The platform 102 can be configured to rotate about an axis of rotation 114 that is central to the cluster of roller tubes 104. For instance, the platform 102 can receive and retain the first, second, third, and fourth roller tubes 104 so that each one is also adjacent the platform's axis of rotation 114 and that each of the roller tube's longitudinal axis 112 is parallel with the axis of rotation 114. In such a configuration, when the platform 102 is rotated, each roller tube 104 revolves around the platform's axis of rotation 114. This is in contrast to each roller tube 104 being rotated about its own longitudinal axis 112, which is how conventional roller bottle systems operate. The platform 102 can include a spindle, hub and bearing assemblies, drive shaft, motors, encoders, sensors, control modules, etc. to facilitate the controlled rotation of the platform 102.

It is contemplated for the platform 102 to be configured to retain each roller tube 104 so that each roller tube's longitudinal axis 112 is parallel with that of the platform's axis of rotation 114. It is further contemplated for the platform's axis of rotation 114 to be parallel with the ground surface so that each roller tube 104 is held on its side when retained in the platform 102. Thus, when a roller tube 104 is retained in the platform 102, its elongated body 110 is parallel with the ground surface such that when media is introduced into the roller tube 104 gravity will cause the media to rest on an inner surface of the roller tube's elongated body 110 portion. As the platform 102 is rotated, media within each roller tube 104 traverses the inner surfaces of the elongated bodies 110 of each roller tube 104 due to the media having a tendency to remain in place by gravity while the roller tube 104 is rotating. As will be explained herein, cells will be caused to adhere to the inner surfaces of each roller tube 104 (in some embodiments the cells are caused to adhere to a cell adherence liner 124 that is removably attached to the inner surfaces of each roller tube 104) along the elongated bodies 110. As the media traverses the inner surfaces, the cells enter into and exit out from the bath of media.

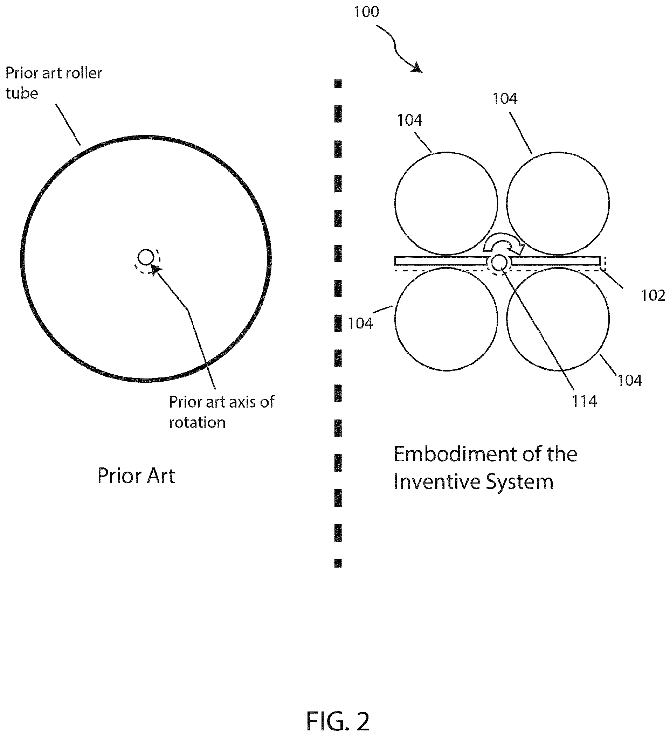

As noted herein, with such an arrangement, it is possible to increase the total circumferential surface area available for cell growth by reducing the size of the roller tubes 104 and increasing the number of roller tubes 104. The combined growth surface area can then be increased without increasing the cubical space requirement. (See FIG. 2). The prior art illustration in the left side of FIG. 2 represents a transverse section of the typical roller bottle revolving around its own longitudinal axis. The exemplary inventive system illustration in the right side of FIG. 2 represents a rotating platform 102 with four smaller roller tubes 104 fixed to the platform 102. As the platform 102 rotates, the smaller roller tubes 104 are rotated 360 degrees but are revolved around axis of rotation 114 of the rotating platform 102.

As the platform 102 rotates, the cells that are adhered to the walls of the roller tubes 104 remain affixed to the roller tubes 104 while the liquid media, because of its attraction to gravity, remains juxtaposed relative to the earth rather than to the rotating platform 102. The effect is to bathe the cells as they rotate through the liquid nutrient medium. The movement of the cells in a clockwise motion, for example, through the liquid medium simulates a counterclockwise sweeping action of the liquid medium over the cells and eliminates cell damaging desiccation of the liquid medium because as vapor is being released into the gaseous space it is simultaneously being swept back into the liquid medium by the rotating action of the cells through the liquid nutrient media.

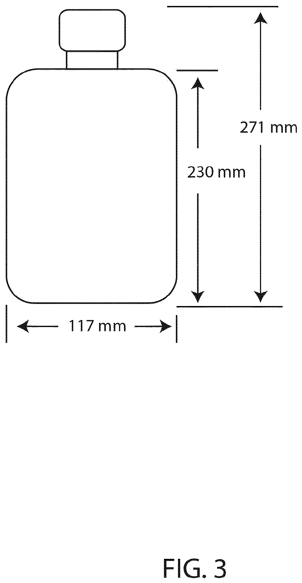

An evaluation of the relative dimensions will demonstrate the increase the total circumferential surface area available without increasing the cubical space requirement. A single roller tube 104 with a diameter of 117 mm will have a circumferential length of 367.57 mm. Each smaller roller tube 104, having a 50 mm diameter, will each have a circumferential length of 157 mm. The combination of four smaller roller tubes 104 will result in a total circumferential length of 628.3 mm, an increase of 1.7 times in total circumferential length while occupying the same cubical space as a 117 mm tube. FIG. 3 shows a schematic representing a 117 mm tube bottle having a total external length of 271 mm. Subtracting the length of the vessel neck and cap, the inside length of the growth surface is 230 mm. Growth surface for cells, in such a 117 mm tube, is 117π x 230 = 84,500 square mm. The total growth surface for four smaller roller tubes 104, each 50 mm in diameter and 271 mm in length, is 4(50π x 271) = 170,274 square mm, which is a 2 fold increase of cell growth surface in less cubical space than occupied by a single 117 mm roller tube.

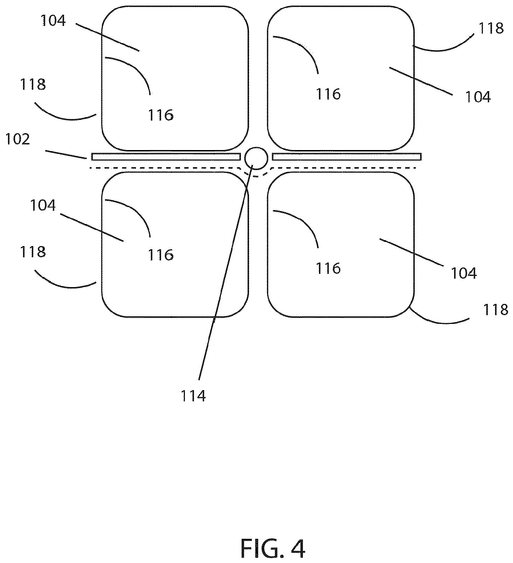

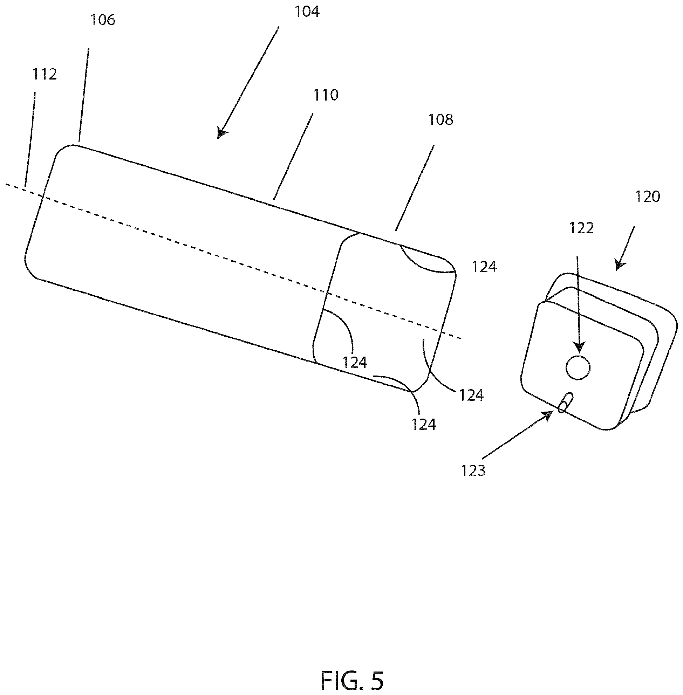

Referring to FIGS. 4-5, as noted herein, each roller tube 104 can have a roller tube first end 106, a roller tube second end 108, and an elongated body 110 with a longitudinal axis 112 running from the roller tube first end 106 to the roller tube second end 108. In some embodiments, the roller tube first end 106 and/or the roller tube second end 108 can be a conjoined portion of the elongated body 110 (i.e., forming a closed end). In some embodiments, the roller tube first end 106 and/or the roller tube second end 108 can be an opening formed in the elongated body 110. In an exemplary embodiment, each of the roller tube first end 106 and the roller tube second end 108 is an opening. The elongated body 110 has a roller tube inner surface 116 and a roller tube outer surface 118. It is contemplated for the roller tube inner surface 116 to be configured to provide a surface to which cells can adhere.

The roller tube 104 can have an end cap 120 that is configured to slidably insert into or on top of the roller tube first end 106 and/or roller tube second end 108. In some embodiments, the roller tube 104 can have a first end cap 120 for the roller tube first end 106, and a second end cap 120 for the roller tube second end 108. The edge of the roller tube first end 106 and/or the edge of the first end cap 120 (or the edge of the roller tube second end 108 and/or the edge of the second end cap 120) can have a seal or gasket to facilitate a fluid seal between the first end cap 120 and the roller tube first end 106 (or the second end cap 120 and the roller tube second end 108) so as to prevent fluid (e.g., gas or liquid) ingress and/or egress to/from the interior of the roller tube 104 via the first end cap-first end interface (or the second end cap-second end interface). For instance, the first end cap 120 (or the second end cap 120) can have a rubber or silicon seal disposed on a circumferential edge that facilitates forming an interference fit and a fluid seal between the end cap 120 and the roller tube end 106, 108 when the end cap 120 is inserted in or on the roller tube end 106, 108. In some embodiments, the end cap 120 can have an inlet/outlet opening 122 to facilitate introduction and/or removal of gas and/or media, and a vent aperture 123 to facilitate venting of fluid (e.g., gas or liquid) from the roller tube 104. These features will be discussed in more detail later.

The roller tube 104 can have a cross sectional shape (a shape exhibited by the roller tube when viewing the roller tube 104 along its longitudinal axis 112). The cross sectional shape can be circular, triangular, square, elliptical, hexagonal, etc. The cross sectional shape of any one roller tube 104 can be the same or different from the cross sectional shape of another roller tube 104. In an exemplary embodiment, the roller tube 104 can have a superellipse cross-sectional shape, which can include a squircle (a shape intermediate between a square and a circle - e.g., a square with rounded edges). (See FIG. 4). This can be done to increase the circumferential surface area available for cell growth within the roller tube 104, as compared to a roller tube 104 having circular cross sectional shape. For instance, a roller tube 104 having a square cross sectional shape with a 12.7 mm radius at each corner will experience an increase in growth area, as compares to a roller tube 104 having a circular cross sectional shape. In addition, four squircle shaped roller tubes 104 each having diameter d would occupy the same cubical space as four circle shaped tubes each having diameter d, but would have an increase in growth area.

Each 50 mm square roller tube 104 with 12.7 mm radius at each corner will have 53,874.8 square mm of growth area. When multiplied by four roller tubes 104 occupying the same cubical space as a single roller tube with a diameter of 117 mm, the cell growth surface becomes 215,499 square mm compared to 84,500 square mm for the single roller tube. That's two and a half times more cell growth surface in the same cubical space. Four roller tubes 104, each 60 mm X 60 mm X 300 mm, would provide 288,000 square mm of total cell growth area versus 84,500 square mm for the single 117 mm roller tube.

The total liquid volume per roller tube 104 would be 1,080,000 cubic mm. The total liquid volume for the single 117 mm tube is 2,000,000 cubic mm. Four roller tubes 104 would contain 4,320,000 cubic mm of liquid. An initial perfusion culture fill (recommended at 10% of total volume) for cell perfusion culturing would be 200 ml for the single 117 mm tube versus 100 ml per smaller roller tube 104 (or 400 ml total). The single 117 mm tube requires 1 ml of nutrient media for each 422.5 square mm of cell growth area. The four smaller tubes 104 requires 1 ml of nutrient media each 687.6 square mm of cell growth area. That's 39% savings in required nutrient media.

The squircle cross sectional shape of the roller tube 104 not only provides an increase in growth surface area, but it also provides a roller tube inner surface 116 ornamentation that is most conducive or salutary for cell growth, as the design maximizes growth surface area while not introducing any sharp corners or ridges that would eliminate or impede the contiguous growth of adherent cells.

While the cells can adhere directly to the roller tube inner surface 116 along the elongated body 110, some embodiments can include a cell adherence liner 124 disposed on the roller tube inner surface 116. The cell adherence liner 124 can be permanently affixed to the inner surface 116 or removably affixed thereto. The cell adherence liner 124 can disposed on a portion of the inner surface 116 or on the entire inner surface 116. With embodiments having a cell adherence liner 124, cells can be caused to adhere directly to the inner surface 116 only (e.g., there may be some portions of the inner surface not covered by the liner), to adhere to the cell adherence liner 124 only, or adhere to both to the inner surface 116 and the cell adherence liner 124.

It is contemplated for the cell adherence liner 124 to be disposed on the entire inner surface 116 (or substantially all of the inner surface 116) so as to prevent cells from adhering directly to the inner surface 116 but rather adhere to the cell adherence liner 124. It is further contemplated for the cell adherence liner 124 to be removable so as to facilitate removal of the cell adherence liner 124 from the roller tube 104 with the cell growth formed on the surface of the cell adherence liner 124. It is contemplated for the cell adherence liner 124 to be configured to stick to the inner surface 116 of the roller tube 104 but be able to be peeled off (e.g., using a user's finger, using forceps, etc.). Once the cell adherence liner 124 (with the cell growth) is removed from the roller tube 104, the cells can be easily collected from the cell adherence liner 124. The cell adherence liner 124 can then be discarded. In some embodiments, the cell adherence liner 124 can be cleaned, sterilized, re-conditioned, etc. and placed back into a roller tube 104 for additional use. The cell adherence liner 124 can be shaped in the form of a tube with a shape profile matching that of the cross sectional shape of the roller tube 104, can be shaped as a plurality of tubular bands each with a shape profile matching that of the cross sectional shape of the roller tube 104, can be shaped as a plurality of elongated strips (e.g., rectangular shaped strips), etc.

The cell adherence liner 124 can have a first liner side surface and a second liner side surface. The first liner side surface can be treated to facilitate cell adherence and growth. The second liner side surface can be configured to exhibit surface attraction to the material (e.g., nonporous polystyrene, nonporous polycarbonate, borosilicate glass, etc.) of which the roller tube 104 is made, can have an adhesive applied thereto, etc. Thus, the cell adherence liner 124 can be configured such that its second liner side surface makes contact with the inner surface 116 of the roller tube 104 to removably attach thereto, and exposed the first liner side surface to the interior cavity of the roller tube 104 to facilitate cell adherence and growth thereon. With such an arrangement, cells and media will come into contact with the first liner side surface, allowing cells to adhere thereto while preventing cells and media from making contact with the inner surface 116 of the roller tube 104 portions that are covered by the cell adherence liner 124.

In an exemplary implementation, a user would remove the end cap(s) 120 and insert the cell adherence liner(s) 124 into the roller tube 104. A user would cause the second liner side surface(s) to make contact with the inner surface 116 of the roller tube 104 so as to allow the cell adherence liner(s) 124 to attach thereto. A user would introduce cells into the roller tube 104 (or the cells can be introduced onto the first liner side surface before the liner(s) 124 are attached to the inner surface 116 of the roller tube 104) so as to allow them to adhere to the first liner side surface of the liner(s) 124. Media can be introduces into the roller tube 104 and the end cap(s) 120 placed on the roller tube first end 106 (or the media can be introduced after the end cap(s) 120 is/are placed on the roller tube first end 106). A cell culture process is then performed with the cells to cause them to grow and be maintained. After the cell culture process is complete, a user would remove the end cap(s) 120 and then remove the liner(s) 124. The cell growth can then be collected from the liner(s) 124.

Embodiments of the cell adherence liner 124 can be made from fluorinated ethylene propylene (FEP) polytetrafluoroethylene (PTFE) (can be referred to as FEP-PTFE, or FEP Teflon ®). The first liner side surface can be corona treated (e.g., using low temperature corona discharge plasma to impart changes in the properties of the surface), plasma etched, etc. to enhance cell attachment thereto. Test results conducted by the inventor demonstrate that the adherence of the FEP-PTFE cell adherence liner 124 second liner side surface to the inner surface 116 of roller tubes 104 made from nonporous polystyrene, nonporous polycarbonate, and borosilicate glass proved to be satisfactory (e.g., exhibits sufficient surface attraction to the roller tube 104 inner surface 116; yet is easy to remove, with cells intact, by lifting away from the roller tube 104 inner surface 116). FEP-PTFE is chemically inert and biocompatible, making it ideal for use in adherent cell cultures.

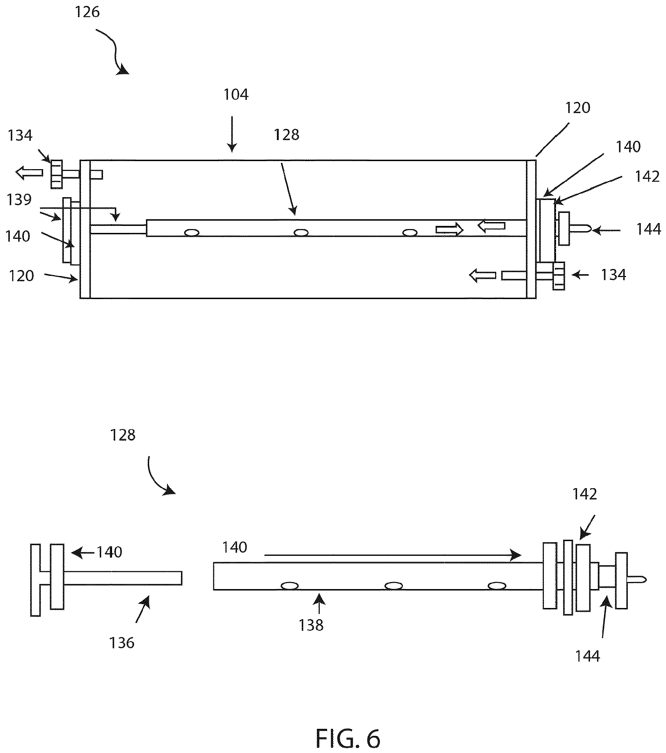

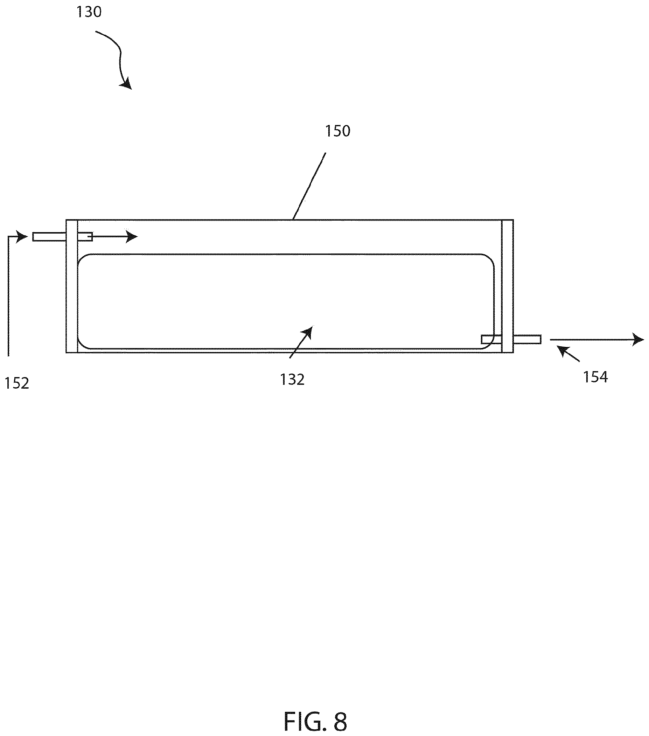

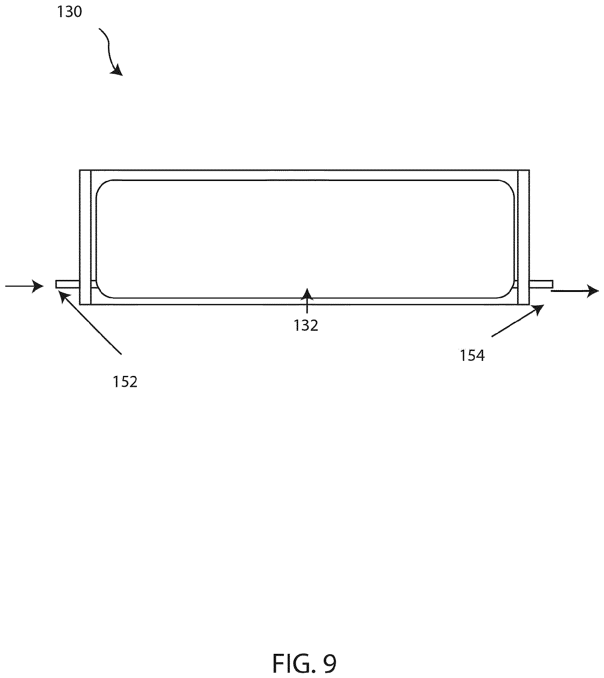

Referring to FIGS. 6-9, some embodiments of the roller tube culture system 100 includes a cell perfusion system 126. The cell perfusion system 126 can be configured to facilitate retention of cells inside the roller tube 104 while simultaneously removing cell waste products and media depleted of nutrients by cell metabolism. The cell perfusion system 126 can be configured to provide fresh media to the cells at a desired or predetermined rate, which can include providing fresh media to the cells at the same rate as the spent media is removed. The cell perfusion system 126 includes a perfusion assembly 128 (e.g., an arrangement of tubing, seals, and valves) within each roller tube 104 that can be placed into fluid communication with a media exchanger tube 130 (e.g., a tube having a bladder 132 or bag that supplies fresh media and a volume of space between the inside of the media exchanger tube 130 and the bladder 132 to receive spent media). Some embodiments of the media exchanger tube 130 can be configured to facilitate co-culturing via trans-mixing. Details of embodiments of the media exchanger tube 130 will be discussed later.

Referring to FIG. 6, the perfusion assembly 128 will be discussed first. As noted herein, the end cap(s) 120 can have at least one inlet/outlet opening 122 to facilitate introduction and/or removal of media. The end cap(s) 120 can also have at least one vent aperture 123 to facilitate venting of gas or liquid from the roller tube 104. The perfusion assembly 128 includes a perfusion tubing arrangement in mechanical connection with each inlet/outlet opening 122 (e.g., an individual perfusion tubing arrangement for each individual inlet/outlet opening 122). The perfusion assembly 128 also includes a perfusion vent assembly 134 in mechanical connection with each vent aperture 123 (e.g., an individual perfusion vent assembly 134 for each individual vent aperture 123). Exemplary embodiments shown in FIG. 6 illustrate the perfusion assembly 128 having a single inlet/outlet opening 122 and two vent apertures 123 formed in the first end cap 120, and a single inlet/outlet opening 122 and two vent apertures 123 formed in the second end cap 120; however, more or less inlet/outlet opening 122 and vent apertures 123 can be used. As will be explained herein, the inlet/outlet openings 122 in each of the first end cap 120 and the second end cap 120 will facilitate support of a perfusion tubing arrangement running along the longitudinal axis 112 of the roller tube 104. It is contemplated for the inlet/outlet openings 122 for each end cap 120 to be located in a central portion of the end caps 120 to facilitate locating the perfusion tubing arrangement within a central portion of the roller tube 104, but the location of the inlet/outlet openings 122 and perfusion tubing arrangement can be at any location. In addition, it is contemplated for the perfusion tubing arrangement to run parallel with the longitudinal axis 112 of the roller tube 104, but it need not to.

The vent aperture(s) 122 can include valves (e.g., check valve) and fittings to facilitate flow of fluid in a desired direction (e.g., flow of fluid into the roller tube 104 and/or out-from the roller tube 104). Thus, the vent aperture 123 can, in addition to providing a venting means, provide a means for perfusion. It is contemplated for the vent aperture(s) 122 of the first end cap 120 to be positioned so that it/they are not subtending the vent aperture(s) 122 of the second end cap 120 - i.e., the vent aperture(s) 122 of the first end cap 120 do not lie directly opposite the vent aperture(s) 122 of the second end cap 120 (e.g., if a geometric line was drawn from a vent aperture 123 of the first end cap 120 to the second end cap 120, that geometric line would not be parallel to the longitudinal axis of the roller tube); however, they can be positioned so as to subtend each other. The purpose of having at least one vent aperture 123 on the first end cap 120 being positioned to not subtend at least one vent aperture 123 on the second end cap 120 is to provide an arrangement in which one of the vent apertures 123 of the first end cap 120 (or second end cap 120) can be submerged in media while at least one of the vent apertures 123 of the second end cap 120 (or first end cap 120) is not submerged in media. As noted earlier, as the roller tube 104 is caused to rotate by the platform 102, the liquid media (because of its attraction to gravity) remains juxtaposed relative to the earth rather than to the rotating platform 102 and remains on a bottom portion of the roller tube 104. Thus, at any given rotational position for a roller tube 104, one of the vent apertures 123 of the first end cap 120 (or second end cap 120) can be submerged in media while at least one of the vent apertures 123 of the second end cap 120 (or first end cap 120) is not submerged in media. This can allow for perfusion via the submerged vent aperture(s) 122 and ventilation via the non-submerged vent aperture(s) 122.

The perfusion tubing arrangement can be at least one tubing 138 configured to controllably release of media or gas into the interior of the roller tube 104. The tubing 138 can be a perforated structure, a semi-permeable membrane, etc. In an exemplary embodiment, the perfusion tubing arrangement can have a shaft 136 and a tubing 138 (e.g., extruded polycarbonate tubing), the tubing 138 being threaded into onto an inert plastic "T" shaped pin 139. The shaft 136 is configured to insert through and mechanically engage with the inlet/outlet openings 122 of an end cap 120. A silicone sealing ring 140 is slipped over the "T" pin 139 so that when the shaft 136 is inserted into and between the end caps 120, the seal 140 is compressed between the shoulder of the "T" pin 139 and the outer face of an end cap 120. The shaft 136 passes through a central tunnel created by the extruded polycarbonate tubing 138 and passes through the inlet/outlet opening 122 of the opposing end cap 120. A second silicone seal 140 is slipped over the tubing 138 that is threaded onto the "T" pin 139 and is pressed between the opposite end cap 120 face and a thin SS washer and SS nut assembly 142 that is threaded onto the tubing 138 via a fitting 144. As the SS nut is tightened, the end caps 120 seal into the tubing 138. Silicone adhesive can be used to seal between the shoulders of the end caps 120 and each end of the roller tube 104.

After assembly of the perfusion tubing arrangement, which functions as a component designed to seal the two end caps 120 in place, the perfusion tubing arrangement, functions as a device to transport liquids and gases from an external source into the roller tube 104. The perfusion tubing arrangement can alternate between functioning as a conduit for the addition of fresh nutrient media for perfusion and for the addition of oxygen containing gas as necessary for the growing cells to convert a carbon source into energy.

The end caps 120, in addition to having an inlet/outlet opening 122 for passage of perfusion tubing arrangement, can have a plurality of vent apertures 123, each capable of being adapted for the transport of liquid or gas into or out of the roller tube 104. As noted earlier, the vent apertures 123 of each end cap 120 can be positioned so as to not subtend each other. For example, with an embodiment in which the first and second end caps 120 are identical and symmetrical, each end cap 120 may be oriented in or on the roller tube end (a roller tube having a squircle cross sectional shape) in any of four positions. The end caps 120 may be oriented with the vent apertures 123 opposed by 180 degrees (see FIG. 6) so that, in use, when one vent aperture 123 is at the bottom-most rotation, and the opposing vent aperture 123 is at the upper-most orientation. Such orientation enables perfusion media to enter via the perfusion tubing arrangement and/or the lower vent aperture 123, while the opposing upper-most vent aperture 123 can function as a vent.

The direction of flow and venting through each vent aperture 123 can be controlled by the placement of a "duck-bill-valve" (e.g., a check valve) at each required location to allow liquid or gas flow in one direction, with a slight positive pressure, and flow restriction in the opposite direction attributed to the designed tension of the duck-bill-valve. FIG. 6 shows end caps 120, each with a single inlet/outlet opening 122; however, a plurality of inlet/outlet openings 122 for each end cap 120 is possible. This may be done to accommodate more advanced culture methods.

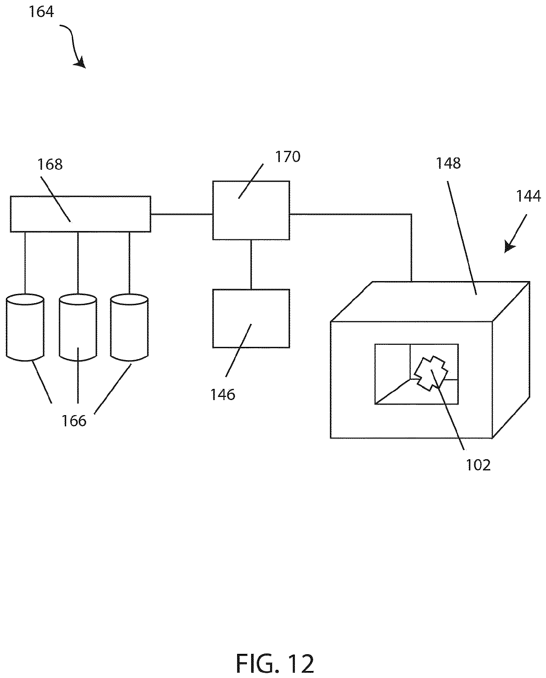

Embodiments of the platform 102 can be used in conjunction with an incubator system 144. (See FIG. 12). The incubator system 144 can include a computer device 146 configured to control aspects of the incubation (e.g., control and monitor rotation of the platform 102, introduction of media, removal of waste, monitor CO₂ and DO levels, etc.) In some embodiments, the incubator system 144 can include a housing 148 and the computer device 146, the housing 148 configured to contain the platform 102 (and the roller tubes 104 placed therein) in a controlled environment. For instance, the housing 148 can provide controlled temperature, humidity, lighting, pressure, etc. via a feedback loop created between various sensors and readers of the system and the computer device to monitor aspects of the incubation in real time and provide control functions to adjust temperature, humidity, lighting, pressure, gas and media flows, CO₂ and DO levels, etc. This can include monitoring conditions within each roller tube 104 individually or in unison, as well as adjusting the temperature, humidity, lighting, pressure, gas and media flows, CO₂ and DO levels, etc. of each roller tube 104 individually or in unison. An example of an incubator system 144 can be the S1-1200 EnviroGenie by Scientific Industries.

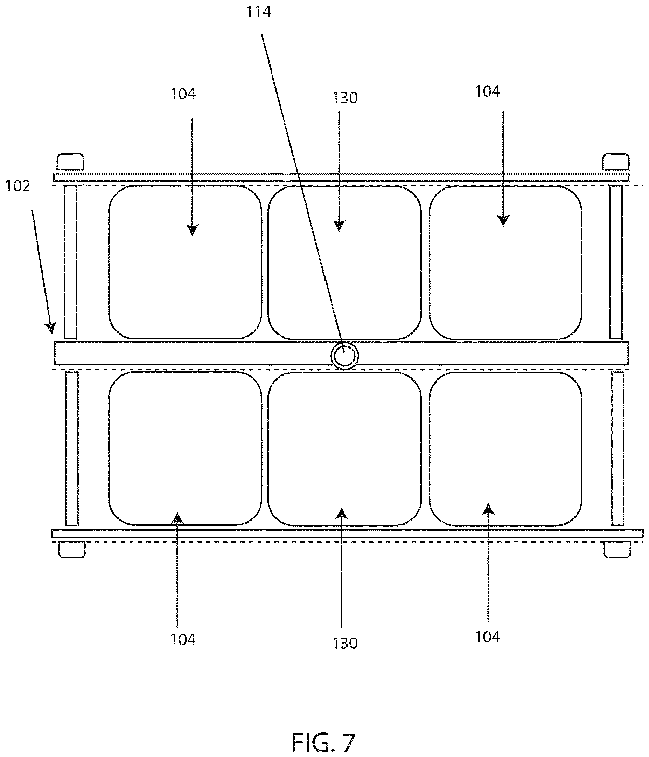

Referring to FIG. 7, embodiments of the incubator system 144 can include at least one media reservoir 130. The media reservoir 130 can be a container used to hold fresh media to be supplied to any one or combination of roller tubes 104. The media reservoir 130 can also be a container used to hold spent media that is removed from any one or combination of roller tubes 104. The introduction and removal of media can be achieved via pumps (e.g., peristatic pump), air compressors, etc. Each pump can be matched for desired electrical requirements, flow rates, and flow pressures so that additional pumps may be utilized as demands increase. It is contemplated for the media reservoirs 130 to be held within the platform 102 so as to allow for rotation of the media reservoirs 130 along with the roller tubes 104. FIG. 7 shows an exemplary embodiment in which the platform 102 is configured to retain four roller tubes 104 and two media reservoirs 130. For instance, the platform 102 can be configured to receive and retain:
- the first roller tube 104 so as to be adjacent the first media reservoir 130, the second media reservoir, and the third media reservoir;
- the first media reservoir 130 so as to be adjacent the first roller tube 104, the second roller tube 104, the third roller tube 104, the second media reservoir 130, and the fourth roller tube 104;
- the second roller tube 104 so as to be adjacent the first media reservoir 130, the second media reservoir 130, and the fourth roller tube 104;
- the third roller tube 104 so as to be adjacent the first roller tube 104, the first media reservoir 130, and the second media reservoir 130;
- the second media reservoir 130 so as to be adjacent the first roller tube 104, the second roller tube 104, the third roller tube 104, the first media reservoir 130, and the fourth roller tube 104; and
- the fourth roller tube 104 so as to be adjacent the first media reservoir 130, the second media reservoir 130, and the second roller tube 104;

Again, the platform 102 can be configured to rotate about an axis of rotation 114 that is central to the cluster of roller tubes 104 and media reservoirs 130. For instance, side surfaces of the first and second media reservoirs 130 (as opposed to the side surface of the first, second, third, and fourth roller tubes 104) can be most proximate to the platform's axis of rotation 114.

Each of the media reservoirs 130 may be identical or different in design. Any combination or permutation of supply or retrieval arrangements can be used. For instance, the first media reservoir 130 may be dedicated for supplying any one or combination of roller tubes 104 with fresh media, while the second media reservoir 130 may be dedicated for receiving spent media from one or combination of roller tubes 104.

Referring to FIG, 8, as another example, the first media reservoir 130 and/or the second media reservoir 130 may be used to both supply fresh media and receive spent media to/from any one or combination of roller tubes 104. This can be achieved via configuring the media reservoir(s) 130 as media exchanger tubes 130. Each media exchanger tube 130 can be a tube-like container 150 having a bladder 132 or bag that supplies fresh media and a volume of space between the inside of the media exchanger tube 130 and the bladder 132 to receive spent media. For instance, the tube-like container 150 can have an inlet nozzle 152 that facilitates introduction of spent media into the volume of space between the inside of the media exchanger tube 130 and the bladder 132. The tube-like container 150 can have an outlet nozzle 154 in fluid connection with the bladder 132 that facilitates supply of fresh media from the bladder 132. This arrangement maintains pressure equilibrium in each media reservoir 130 - e.g., as the bladder(s) 132 empty, spent media from the roller tube(s) 104 occupies the void left by media pumping from the bladder(s) 132. In an exemplary embodiment, the first media exchanger tube 130 can be configured to supply fresh media to the first and second roller tubes 104, as well as receive spent media from the first and second roller tubes 104. The second media exchanger tube 130 can be configured to supply fresh media to the third and fourth roller tubes 104, as well as receive spent media from the third and fourth roller tubes 104.

In an exemplary implementation, a bladder 132 containing 1 L of fresh media can be loaded into a tube-like container 150. The bladder 132 can be connected to a battery powered peristaltic pump. Perfusion occurs as peristaltic pump(s) direct fresh nutrient media to the roller tube(s) 104. A flow rate of between 0.75 and 1.5 ml per minute can be used to add fresh media to the initial charge of 100 ml of media and seed culture in each roller tube 104. Tubing from each peristaltic pump may be connected through a liquid distribution system of tubing for delivering liquid media to and from the roller tube(s) 104 through a selection of liquid entry and exit ports (e.g., inlet/outlet openings 122 and/or vent apertures 123), as may be required for performing advanced culture methods. The pumps can stop when 50 ml of fresh media has been added (e.g., in just over 33 minutes) to the roller tube(s) 104. At the end of 24 hours, a separate peristaltic pump can be activated to remove 50 ml of spent media from the roller tube(s) 104, and return the spent media to its corresponding media exchanger tube 130. The rotation (e.g., 1 rpm) of the platform 102 can be stopped for spent media removal, and the rotating can be resumed while an equivalent amount of fresh media is pumped into the roller tube(s) 104 to replace the spent media that was removed. The rotation of the platform 102 can continue at 1 rpms so that perfusion feeding continues. This can be done for 24 hours, for example. The perfusion and spent media removal can continue until the fresh media bladder 132 is empty and has been replaced by an equal volume of spent media (e.g., at the end of ten days). A media exchanger tube 130 containing fresh media (or a bladder 132 containing fresh media) can be easily exchanged or replaced. Perfusion can then continue (e.g., for an additional ten days).

As noted above, embodiments of the media exchanger tube 130 can be configured to facilitate co-culturing via trans-mixing. Cell to cell signaling is an area of cell science that will require many hours, months, and years of research. For instance, breast cancer tissue contain carcinoma cells and stromal cells. Intratumoral stroma is composed of various types of cells (e.g., fibroblasts, adipocytes, inflammatory lymphocytes, macrophages, lymphatic cells and blood capillary cells (including pericytes and endothelial cells), etc.). Communications and interactions among these cells play an important role in cancer initiation, promotion, and progression through cytokine signaling. Non-automated co-culturing methods are very time consuming and prone to contamination. Embodiments of the roller tube culture system 100, however, can configured to facilitate co-culturing methods via a closed loop automation system by growing different cells in adjacent roller tubes 104, and then trans-mixing the secretions from the different cells so that cytokines etc. may communicate with each companion mono-cell culture.

With co-culturing, any one or combination of the media exchanger tubes 130 can be configured so that spent media is returned to the fresh media bladder 132 for trans-mixing of media from the active perfusion culture roller tube(s) 104. Thus, the inlet nozzle 152 and the outlet nozzle 154 of the media exchanger tube 130 can both be in fluid connection with the bladder 132, allowing for the trans-mixing. The media returning from a co-culture growth roller tube 104 (e.g., the first roller tube 104) is trans-mixed with fresh media in a media exchanger tube 130 until co-cultures, in a separate roller tube 104, are exposed to cytokines from each co-culture. With the roller tube culture system 100, the entire process can be a closed-loop, and the perfusion cultures remain separate, in independent growth reactors (e.g., in independent roller tubes 104), while cell signal communication is established by and through the trans-mixed secretions from each separate perfusion culture.

The pumps, air compressors, etc. can be battery operated and mounted onto the rotatable platform 102 such that there are no wires or tubing connections running to and/or from the platform 102. The system can be configured as close-looped, and closed to external sources for potential contamination.

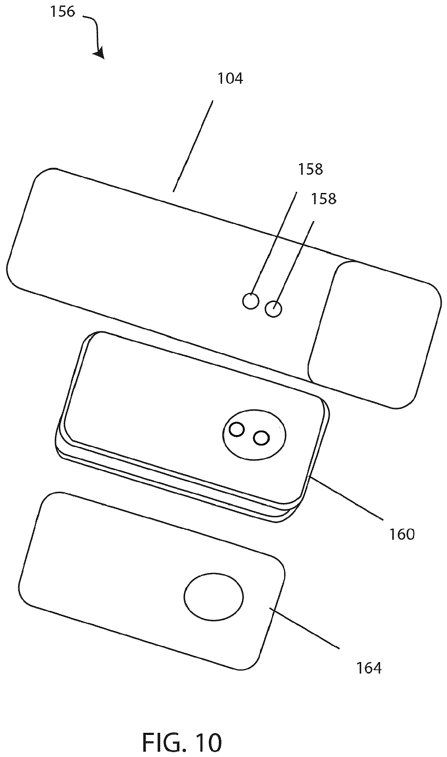

Referring to FIG. 10, embodiments of the roller tube culture system 100 can include a cell culture monitoring system 156. The cell culture monitoring system 156 includes at least one sensor patch 158 and a reader 160 to facilitate real-time pH, CO₂, and/or DO monitoring. The cell culture monitoring system 156 can include any embodiment disclosed U.S. 10,379,047, which is incorporated herein by reference in its entirety. Each sensor patch 158 can include a biocompatible silicone adhesive backing so as to facilitate attachment to a surface of the roller tube 104 and/or the cell adherence liner 124. In some embodiments, the sensor patches 158 can be placed such that they come into contact with the liquid media inside the roller tube 104. As explained in greater detail in U.S. 10,379,047, the senor patches 158 can be excited by light transmitted through the roller tube 104, and respond with luminescence that is detected by a reader 160 and interpreted quantitatively. In an exemplary implementation, the reader 160 generates excitation light beams that are collimated and directed into the roller tube(s) 104 so as to be incident upon the sensor patch(s) 158. Illumination sources (e.g., light emitting diodes) in the reader 160 provide excitation source for fluorophores impregnated into each sensor patch 158 to emit specific fluorescent light frequencies in response. The intensity of the response signals are detected and interpreted by firmware in the reader 160 and/or are transmitted to the computer device 146 for interpretation.

Some embodiments can include an alignment device 162 configured to align the reader 160 with the sensor patch(es) 158. For instance, the alignment device 162 can include a mat that is approximately 3 mm thick, the mat being die-cut to match and adhere to a top surface of the reader 160. In an exemplary embodiment, reader 160 is externally 58 mm wide and approximately 100 mm in length but may be approximately 200 mm in length, depending on the size of the contained battery pack used for powering the device 160. The circuitry that conditions the signal from the excitation light sources is mounted on a circuit board that is 35 mm square. The reader 160 comprises a fixed wavelength mini fluorometer with two light emitting diodes (LED's) fixed at 403 nm (violet) and 460 nm (blue) as excitation sources. The LED's are situated and directed into each of two beam combiner housings that are mounted side by side on the 35 mm square circuit board. The light from the LED's is collimated in each beam combiner and passes through a series of filters and dichroic mirrors so that the conditioned light signals emit in parallel from the beam combiners. The collimated light beams are 3 mm in width at 25 mm from the point of emission. The centers of the parallel collimated beams are 18 mm apart. The 35 mm square circuit board, with the beam combiners affixed, is mounted within the housing such that the emitting beams will be coincident with the central long axis of the housing. In an exemplary embodiment, the fluorescent sensor patches 158, each more or less being 9 mm in diameter, are mounted within the roller tube 104, and are affixed onto the inner wall of the roller tube 104 coincident with the long axis of the roller tube 104 and 18 mm from center to center such that they will align, longitudinally and distally, with the collimated excitation light beams of the reader 160 when it is affixed onto the external surface of the roller tube 104. If a cell adherence liner 124 is to be used in the culture process, the liner 124 is assembled first within the roller tube 104 and the sensor patches 158 are mounted onto the liner 124 so that the sensor surfaces are in contact with the growing cells and liquid medium within the roller tube 104. The sensor patches 158 are mounted with a biocompatible silicone adhesive that will adhere to the treated surface of the liner 124. The entire assembled roller tube 104 may then be packaged and sterilized for use.

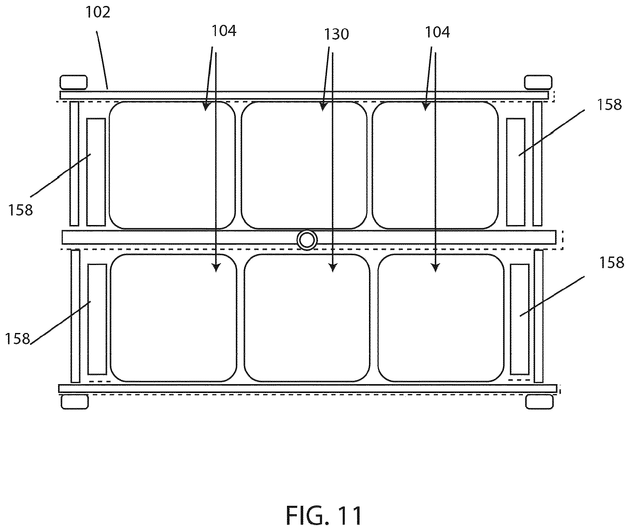

FIG. 11 illustrates an exemplary embodiment of how the cell culture monitoring system 156 can be used with an embodiment of the platform 102 so as to not interfere with, nor be influenced by, the rotating action of the roller tube(s) 104.

Referring to FIG. 12, some embodiments of the incubator system 144 can have a gas circulation system 164 configured to adjust the CO₂ and dissolved O₂ levels in the roller tube(s) 104. Instead of replacing the media when it is detected that the CO₂ and dissolved O₂ levels are trending to undesirable levels, the levels of CO₂ and dissolved O₂ can be adjusted. This can be achieved by the gas circulation system 164 introducing and/or removing gas from the incubation environment of the roller tube(s) 104. The introduction and/or removal of gas can include gas suitable for cell growth, such as oxygen, carbon dioxide, nitrogen, or a mixture of these gases. For instance, a gas supply 166 (e.g., a gas tank) can be equipped with pumps, compressors, line or hoses, valves, couplings, regulators, etc. to provide a pressurized system (e.g., positive pressure) that is a gas reservoir in fluid communication with the roller tube(s) 104 and further configured to introduce gas to the roller tube(s) 104. The fluid communication between the gas supply 166 and the roller tube(s) 104 can be achieved via the perfusion assembly 128.

The gas circulation system 164 can include a gas manifold 168 and/or multiplexer 170 configured to facilitate introducing and/or removing gas to and from any one or combination of the plurality of the roller tube(s) 104. Other components such as filters, humidifiers, heaters, etc. can be used to purify and /or condition the gas before it is introduced into the roller tube(s) 104. The gas in all of the roller tubes 104 Is can be adjusted in unison, the gas in any one or combination of the roller tubes 104 can be adjusted at different rates, concentrations, volumes, etc.

Some embodiments can include a discharge tank equipped with pumps, compressors, line or hoses, valves, couplings, regulators, etc. to provide a pressurized system (e.g., negative pressure) that is a gas reservoir in fluid communication with the roller tubes 104 and further configured for receiving gas from the roller tubes 104. This can facilitate removal of gas from a roller tube 104.

The gas circulation system 164 can be configured for supplying the roller tube(s) 104 with the proper gas(es) for optimum cell growth. For instance, the gas circulation system 164 can include a first gas supply 166 (e.g., a CO₂ gas tank), a second gas supply 166 (e.g., a dissolved O₂ tank), a third gas supply 166 (e.g., a nitrogen gas tank), etc. More of less gas supplies 166 and other gases can be used. Each gas supply 166 can be connected to the incubator system 144 so that each gas supply 166 is connected to each roller tube 104 via the gas manifold 168 and/or multiplexer 170. Supply lines or hoses from each gas supply 166 transports the gas to the manifold 168 and/or multiplexer 170 where the gases can be directed to a particular roller tube 104 and/or blended together as desired for a particular application (after mixing, the mixed gas can then be transported to any one or combination of roller tubes 104).

In one embodiment, the incubator system 144 includes a housing 148 with a lid. The lid can be hingeldly attached to the housing 148. In some embodiments, the lid can include a seal or gasket to provide a fluid-tight seal between the lid and the housing 148. The housing 148 can define a volume of space within which the platform 102 is held. The incubator system 144 can include the gas manifold 168 and/or the multiplexer 170 to facilitate directional flow of the gas from the gas source(s) 166 to the roller tube(s) 104. In some embodiments, the housing 148 can be configured to provide a positive pressure within the volume of space so as to inhibit or prevent unwanted gases from entering the housing 148 and possibly entering the incubation environment of the roller tube(s) 104. The housing 148 can also be equipped with a heater, a humidifier, lighting, etc. to provide a conditioned environment for the cell cultures within the roller tube(s) 104 placed therein.

It will be apparent to those skilled in the art that numerous modifications and variations of the described examples and embodiments are possible in light of the above teachings of the disclosure. The disclosed examples and embodiments are presented for purposes of illustration only. Other alternate embodiments may include some or all of the features disclosed herein. Therefore, it is the intent to cover all such modifications and alternate embodiments as may come within the true scope of this invention, which is to be given the full breadth thereof. Additionally, the disclosure of a range of values is a disclosure of every numerical value within that range, including the end points.

## Claims

1. A platform for a cell culture system, comprising:
a framework configured to rotate about an axis of rotation; and
the framework configured to receive and retain a plurality of reaction vessels so as to arrange each reaction vessels adjacent to the axis of rotation.

2. The platform of claim 1, wherein:
the framework is configured to arrange the plurality of reaction vessels circumferentially about the axis of rotation; and/or
the framework is configured to arrange the plurality of reaction vessels so that a longitudinal axis of each reaction vessel is parallel with the axis of rotation.

3. A platform for a cell culture system, comprising:
a framework configured to rotate about an axis of rotation; and
the framework configured to receive and retain a plurality of reaction vessels so as to cluster the plurality of reaction vessels around the axis of rotation.

4. The platform of claim 3, wherein:
when the framework is rotated about the axis of rotation, the plurality of reaction vessels revolves about the axis of rotation;
the framework is further configured to receive and retain at least one media reservoir; and/or
the framework is further configured to cluster the plurality of reaction vessels and the at least one media reservoir around the axis of rotation.

5. The platform of claim 4, wherein:
when the framework is rotated about the axis of rotation, the plurality of reaction vessels and the at least one media reservoir revolve about the axis of rotation.

6. A reaction vessel, comprising:
a roller tube having a roller tube first end, a roller tube second end, and an elongated body;
wherein the roller tube has a superellipse cross sectional shape.

7. A reaction vessel, comprising:
a roller tube having a roller tube first end, a roller tube second end, and an elongated body;
a first end cap configured to be removably attached to the roller tube first end, the first end cap having a first inlet/outlet opening and a first vent aperture;
a second end cap configured to be removably attached to the roller tube second end, the second end cap having second inlet/outlet opening and a second vent aperture; and
tubing extending from the first inlet/outlet opening to the second inlet/outlet opening, the tubing configured to controllably release media and/or gas.

8. A cell adherence liner for a reaction vessel, comprising:
a liner having a liner first side and a liner second side;
the liner first side being treated to facilitate cell adherence and cell growth; and
the liner second side being configured to exhibit surface attraction to material of the reaction vessel to facilitate removable attachment to the reaction vessel and/or having an adhesive applied thereto to facilitate removable attachment to the reaction vessel.

9. The cell adherence liner of claim 8, wherein:
the liner first side is corona treated and/or plasma etched; or
the liner is fluorinated ethylene propylene (FEP) polytetrafluoroethylene (PTFE).

10. A media exchanger vessel, comprising:
a container having a cavity and a bladder within the cavity;
a volume of space between an inner surface of the container and the bladder;
an inlet nozzle configured to facilitate introduction of spent media into the container;
an outlet nozzle configured to facilitate flow of fresh media from the bladder.

11. The media exchanger vessel of claim 10, wherein:
the inlet nozzle is in fluid communication with the volume of space between the inner surface of the container and the bladder; or
the inlet nozzle is in fluid communication with the bladder.

12. A roller tube culture system, comprising:
a rotating platform, comprising:
a framework configured to rotate about an axis of rotation; and
the framework configured to receive and retain a plurality of reaction vessels and at least one media reservoir so as to cluster the plurality of reaction vessels and the at least one media reservoir around the axis of rotation;
each reaction vessel comprises:
a roller tube having a roller tube first end, a roller tube second end, and an elongated body, wherein the roller tube has a superellipse cross sectional shape;
a first end cap configured to be removably attached to the roller tube first end, the first end cap having a first inlet/outlet opening and a first vent aperture;
a second end cap configured to be removably attached to the roller tube second end, the second end cap having second inlet/outlet opening and a second vent aperture; and
tubing extending from the first inlet/outlet opening to the second inlet/outlet opening, the tubing configured to controllably release media and/or gas;
a cell adherence liner, comprising:
a liner having a liner first side and a liner second side;
the liner first side being treated to facilitate cell adherence and cell growth; and
the liner second side being configured to exhibit surface attraction to material of the reaction vessel to facilitate removable attachment to the reaction vessel and/or having an adhesive applied thereto to facilitate removable attachment to the reaction vessel;
the at least one media reservoir comprises:
a container having a cavity and a bladder within the cavity;
a volume of space between an inner surface of the container and the bladder;
an inlet nozzle configured to facilitate introduction of spent media into the container; and
an outlet nozzle configured to facilitate flow of fresh media from the bladder.

13. The roller tube culture system of claim 12, further comprising at least one of:
a cell culture monitoring system;
a gas circulation system;
a cell perfusion system; or
a co-culturing system.
